# EUROPEAN PATENT APPLICATION

(11) **EP 4 707 397 A1**
(43) Date of publication of application: **11.03.2026**
(21) Application number: 24797484.3
(22) Date of filing: 26.04.2024
(51) Int. Cl.: C12N 15/86, C12N 5/0793

(54) **PROMOTER FOR GENE EXPRESSION IN RETINAL CELLS, AND VECTOR SYSTEM COMPRISING SAME**

(30) Priority: 27.04.2023 KR 20230055573
(71) Applicant: Korea Research Institute of Bioscience and Biotechnology, Daejeon 34141 (KR)
(72) Inventor: CHUNG, Kyung-Sook, Daejeon 34141 (KR); JANG, Hyeon Ki, Daejeon 34141 (KR); KWON, Jae-eun, Daejeon 34141 (KR); KIM, Seung-Hyun, Daejeon 34141 (KR); KWON, Ok Seon, Daejeon 34141 (KR); AHN, Jiwon, Daejeon 34141 (KR); SON, Myung Jin, Daejeon 34141 (KR)
(74) Representative: Symbiosis IP Limited
(86) International application number: PCT/KR2024/005706
(87) International publication number: WO 2024/225830

(57) **Abstract**

The present invention relates to a promoter for gene expression in retinal cells, and a vector system comprising same. The promoter for gene expression in retinal cells and the vector system including same according to the present invention have a minimal unit for promoter activity and, unlike existing known promoters in retinal cells, maintain a high level of expression of a target gene, and thus can be used to maximize the expression of the target protein in retinal cells.

## Description

### [Technical Field]

The present disclosure relates to a promoter for gene expression in retinal cells and a vector system comprising the same.

### [Background Art]

Retinal degeneration-related diseases are promising targets for therapeutic development using adeno-associated virus (AAV)-mediated gene therapy. AAV vectors may mediate long-term gene expression in the retina and induce a minimal immune response, and thus the potential for gene therapy using these vectors is very high. The retina is the light-sensitive tissue at the back of the eye composed of a variety of cell types, including retinal cells, photoreceptor cells, retinal pigment epithelial cells, retinal ganglion cells, and the like. The target cell type and vector delivery pathway for AAV gene therapy vectors may have a large impact on disease indications. For example, a phase 1 clinical trial for age-related macular degeneration uses intravitreal delivery of vectors to achieve transduction of retinal ganglion cells, and a recent clinical trial for the treatment of patients with Leber congenital amaurosis type 2, a form of retinitis pigmentosa, uses subretinal delivery of the RPE65 gene to transduce into retinal pigment epithelial cells.

In light of its utility, there is a need to develop novel agents and methods for improving AAV delivery to the eye.

However, the development of therapeutics related to this is progressing very slowly. In other words, even though one of the approaches capable of treating a disease is gene therapy (particularly, AAV-based gene therapy), therapeutic approaches are largely limited in two aspects.

First, in the case of AAV-based gene therapy for direct application to cells through the retina, the size of the gene that can be used is very limited. Specifically, AAV has a very limited design aspect of the vector because AAV has a very limited packaging capacity of 4.7 kb and cannot accommodate endogenous promoters, which are typically several kb in length.

Second, there are various cells in the retina as described above, and among them, the cells that cause the disease may be limited to specific cells. That is, the type of cells requiring genetic correction or treatment is specified for the treatment of diseases, and the therapeutic agent should operate on such cells, but general AAV-based techniques used up to now do not exhibit sufficient efficacy in terms of tissue specificity.

Specifically, since it is very difficult to design a vector including a suitable promoter, enhancer, and the like within a small size range of 4.7 kb, which is the packaging capacity of AAV, and to allow the vector to have sufficient efficacy by overexpressing a target protein specifically in a target cell, the gene therapy methods developed up to now use universal promoters expressed in all cells and therapeutic agents that specifically express genes in target cells may be considered to be extremely limited. Expression in target cells is important as a method for minimizing the side effects of the gene therapy agent.

Under this background, the present inventors have developed a novel retinal cell-specific and minimized-size promoter and a vector system including the same, thereby completing the present disclosure.

### [Disclosure]

### [Technical Problem]

An object of the present disclosure is to provide a promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7.

Another object of the present disclosure is to provide an expression cassette comprising: a promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7.

Still another object of the present disclosure is to provide an expression cassette comprising: a. a promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7; and

b. any one enhancer selected from the group consisting of SEQ ID NOs: 12 to 18 and 28 to 31.

Still another object of the present disclosure is to provide a vector comprising the promoter and/or the expression cassette.

Still another object of the present disclosure is to provide an adeno-associated virus (AAV) vector comprising the promoter and/or the expression cassette.

Still another object of the present disclosure is to provide a cell transformed with the expression cassette or the vector comprising the expression cassette.

Still another object of the present disclosure is to provide a pharmaceutical composition comprising the expression cassette or the vector comprising the expression cassette; and a pharmaceutically acceptable excipient.

Still another object of the present disclosure is to provide a pharmaceutical composition for preventing or treating an eye disease, comprising the expression cassette or the vector comprising the expression cassette.

Still another object of the present disclosure is to provide a use of the expression cassette or the vector comprising the same for treating an eye disease.

Still another object of the present disclosure is to provide a method for treating an eye disease comprising administering the expression cassette or the vector comprising the same to a subject in need thereof.

Still another object of the present disclosure is to provide a method for enhancing expression of a target gene in the retina, comprising administering the expression cassette or the vector comprising the same to a subject in need thereof.

### [Technical Solution]

The present disclosure provides a novel promoter having a short length to be suitable for use in a vector system, such as a viral vector system, particularly an AAV vector system, and being capable of specifically inducing gene expression in retinal cells at a high level, and an expression cassette comprising the same.

The term "promoter" refers to a regulatory element that directs transcription of an operably linked nucleic acid. The promoter may regulate both the transcription rate and efficiency of the operably linked nucleic acid. The promoter may also be operably linked to other regulatory elements that enhance ("enhancer") or repress ("repressor") promoter-dependent transcription of a nucleic acid. "Promoter activity" refers to the ability of a promoter to initiate transcription of an operably linked nucleic acid.

The phrase "operably linked" means that nucleic acid sequences are associated on a single nucleic acid molecule, so that one action is affected by the other. For example, a promoter is operably linked to a coding sequence if it can affect the expression of the coding sequence, i.e., if the coding sequence is under the transcriptional control of the promoter.

Accordingly, the present disclosure provides a promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7.

More specifically, the present disclosure provides a retinal cell-specific promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7.

Accordingly, the present disclosure provides a sequence having a nucleotide sequence that has at least 90% identity to the sequence of SEQ ID NO: 6 or SEQ ID NO: 7.

In the present disclosure, retinal cells collectively refer to cells constituting the retina. Retinal cells include photoreceptor cells, retinal ganglion cells, Muller cells, bipolar cells, amacrine cells, horizontal cells, and/or retinal pigment epithelial cells, and the like, and the retina is formed around these cells.

The promoter according to the present disclosure is a promoter that specifically acts on retinal cells and may show a high expression level in retinal cells. More specifically, the promoter may exhibit high expression specifically for photoreceptor cells.

The promoter of the present disclosure is a retinal-specific promoter which exhibits a relevant activity and, when introduced into a retinal cell, may initiate transcription of a nucleic acid operably linked thereto at a high expression level. "Retinal-specific" will be understood to mean a promoter that is mainly active in retinal cells. It should be understood that generally lower expression within other tissues or cells cannot be excluded entirely.

The promoter of SEQ ID NO: 6 or SEQ ID NO: 7 according to the present disclosure is a promoter sequence that maintains tissue specificity and high expression while reducing the size to a certain level or more compared to the existing RS1 promoter sequence known to be photoreceptor-specifically used. Having a shorter promoter sequence is advantageous because it allows the overall size (or length) to be reduced in the vector, particularly in the rAAV genome. This enables the production of rAAV vectors with more efficiently packaged genomes.

More specifically, the sequence of SEQ ID NO: 6 according to the present disclosure is a partial region of the GRK1 promoter sequence. More specifically, when compared to the known GRK1 promoter region, the sequence may exhibit high tissue specificity and high expression level while having a total of 215 short sequences.

The sequence of SEQ ID NO: 7 according to the present disclosure is a partial region of the PDE6B promoter sequence. More specifically, when compared to the known PDE6B promoter region, the sequence may exhibit high tissue specificity and high expression level while having a total of 114 short sequences.

As used herein, at least 90% identity means having at least 90, 91, 92, 93, 94, 95, 96, 97, 98, 99% or 100% identity.

Specifically, the promoter of the present disclosure may be a functional variant having at least 90, 91, 92, 93, 94, 95, 96, 97, 98, 99% or 100% identity to the sequence of SEQ ID NO: 6 or SEQ ID NO: 7. The phrase "sequence identity" refers to the number (%) of matches (nucleic acid residues with homogeneity) at positions from the alignment of the two nucleotide sequences. Sequence identity is measured by comparing sequences when aligned to maximize overlap and homology while minimizing sequence gaps. In particular, sequence identity may be measured using any of a number of mathematical whole or local alignment algorithms, depending on the length of the two sequences.

Functional variants refer to nucleotide sequences that retain their essential properties. In general, variants are very similar overall and are identical to the original nucleotides in many regions. The sequence of the variant may differ by nucleotide substitution, deletion or insertion of one or more nucleotides in the sequence, which does not impair promoter activity. Variants may have the same length as the original sequence, or may be shorter or longer.

As used herein, the term "core" region (which may also be referred to herein as "core sequence" or "core nucleotide sequence" or "consensus region" or "consensus nucleotide sequence") refers to a central sequence capable of specifically inducing gene expression within retinal cells at a high level. A "promoter comprising a core nucleotide sequence" should be construed to mean that the promoter has at least a core nucleotide sequence, but may include additional components, such as additional nucleotide sequences.

If slightly extended by inclusion of additional nucleotides (e.g., by generating what is defined herein as an "extended core" region), it may be referred to as an "extended core region" or "extended core sequence" or "extended core nucleotide sequence", etc.

Accordingly, there is provided a promoter of 100 to 400 nucleotides in length comprising the sequence of SEQ ID NO: 6 or SEQ ID NO: 7 as a core nucleotide sequence. Here, the core nucleotide sequence is understood to include the above-described sequence of SEQ ID NO: 6 or 7 as it is or to include some additional extension sequences in individual sequences, but include all nucleotides of the sequence.

The number of these sequences is not limited, but additional sequences may be included at the 3' and 5' ends as long as the number of nucleotides is 400 nucleotides or less to provide a minimum length vector for use with AAV vectors, etc. The number of any additional possible nucleotides described above includes all of any integer capable of being set within the number of total length 400 or less described above.

In other words, based on each GRK1 promoter or PDE6B promoter sequence, it may further comprise at least 10, at least 15, or at least 20 consecutive nucleotides at the 3' end of each of the above-described sequences. In addition, based on each GRK1 promoter or PDE6B promoter sequence, it may further comprise at least 10, at least 15, or at least 20 consecutive nucleotides at the 5' end of each of the above-described sequences.

The promoter of 100 to 400 nucleotides in length described above may preferably comprise 100 to 350, more preferably 110 to 300 nucleotides. Even more preferably, the length may be greater than or equal to the minimum number and less than 400 of sequences of each of SEQ ID NO: 6 or SEQ ID NO: 7. Further, the length may be greater than or equal to the minimum number and less than 350 of sequences of SEQ ID NO: 6 or SEQ ID NO: 7. In addition, the length may be greater than or equal to the minimum number and less than 300 of sequences of SEQ ID NO: 6 or SEQ ID NO: 7.

More preferably, the promoter region of SEQ ID NO: 6 or SEQ ID NO: 7 may further include a 5'UTR region at the 5' end. Specifically, the UTR sequence may be a sequence located upstream (5' UTR) of the GRK1 promoter or PDE6B promoter found in the human genomic sequence or a portion thereof. More specifically, the UTR sequence may have a length of at least 1 bp, 2 bp, 3 bp, 4 bp, 5 bp, 6 bp, 7 bp, 8 bp, 9 bp, 10 bp, 20 bp, 30 bp, 40 bp, 50 bp, 60 bp, 70bp, 80 bp, 90 bp, 100 bp, 200 bp, 300 bp, 400 bp, 500 bp, 600 bp, 700 bp, 800 bp, 900 bp, 1 Kb, 2 Kb, 3 Kb, 4 Kb, 5 Kb, 6 Kb, 7 Kb, 8 Kb, 9 Kb, or 10 Kb.

More preferably, the 5' UTR may be optimized for the desired protein expression level. The optimized sequence may have, for example, a nucleotide sequence of SEQ ID NO: 8, SEQ ID NO: 9, or SEQ ID NO: 24, but is not limited thereto.

SEQ ID NO: 8 is preferred as the 5'UTR region for GRK1 promoter.

SEQ ID NO: 9 is preferred as the 5'UTR region for PDE6B promoter.

The addition of this 5' UTR confers improved efficacy in terms of expression efficacy as well as improved efficacy in terms of tissue specificity.

The present disclosure provides an expression cassette comprising: a promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7; and a nucleotide encoding a polypeptide operably linked thereto.

The term "expression cassette" refers to a nucleic acid construct including a coding sequence and one or more sequences required for expression of the coding sequence. In particular, one of these regulatory sequences is the promoter of the present disclosure.

The expression cassette comprises a coding sequence and regulatory sequences both upstream (5' untranslated region) and downstream (3' untranslated region) of the coding sequence that are required for the expression of the selected gene product. Thus, the expression cassette typically comprises a promoter sequence, a coding sequence and a 3' untranslated region that generally contains a polyadenylation site and/or a transcription terminator. The expression cassette may also contain additional regulatory elements such as, for example, enhancer sequences, polylinker sequences that facilitate insertion of the DNA fragment into the vector and/or splicing the signal sequence. The expression cassettes are generally contained within the vector to facilitate cloning and transformation.

The promoter of the present disclosure is operably linked to a heterologous nucleic acid. As used herein, the term "heterologous" refers to a nucleic acid other than a nucleic acid to which a promoter is operably linked within a naturally occurring genome.

In an embodiment, a nucleotide encoding a polypeptide operably linked to the promoter of the present disclosure encodes the desired polypeptide. The desired polypeptide may be any polypeptide required to be expressed in retinal cells.

The desired polypeptide may be a therapeutic polypeptide or a reporter protein.

The desired gene may be any gene targeted for expression in the retina, preferably in photoreceptor cells.

In an embodiment, the nucleic acid operably linked to the promoter of the present disclosure is a gene of interest, preferably a therapeutic gene, encoding a therapeutic polypeptide.

A target gene, preferably a therapeutic gene, refers to a gene encoding a therapeutic protein useful for the treatment of a pathological condition. A therapeutic gene, when expressed, confers a beneficial effect in the cell or tissue in which it is present, or in the patient in which the gene is expressed. Examples of advantageous effects include alleviation of a signal or symptom of a condition or disease, prevention or inhibition of a condition or disease, or conferment of desirable properties. Therapeutic genes include genes that partially or wholly correct genetic defects in a patient. In particular, the therapeutic gene may be a nucleic acid sequence encoding a protein useful in gene therapy that alleviates a defect caused by a deletion or defect of the protein in a cell or tissue of a subject, but is not limited thereto.

Examples of therapeutic genes include, but are not limited to, RS1, RP1, Rho, Rom1, SPATA7, PCARE, CRB1, IMPG1, GNAT1, RPGRIP1, OPN1SW, TULP1, ABCA4, GUCA1A, CNGB1, GUCA1B, PRCD, RGR, CDHR1, RP1L:, RBP3, PRPH2, IMPG2, PDE6B, RD3, GNB1, OPN1LW, OPN1MW, MYO7A, MAK, USH1C, PROM1, CRX, GUCY2D, NR2E3, OPN3, OPN4, IQCB1, and/or GNGT1.

The reporter protein is detectable in living retinal cells. The expression of the reporter protein under the control of the promoter of the present disclosure allows the retinal cells to be specifically detected or identified. The reporter protein may be, for example, a fluorescent protein (e.g., GFP), a calcium indicator (e.g., GCaMP), luciferase, alkaline phosphatase, beta-galactosidase, beta-lactamase, horseradish peroxidase, mCherry, or variants thereof.

The expression cassette according to the present disclosure may include one or more untranslated regions (UTRs) or sequences, and more preferably, may further include 5'UTR sequences. The UTR sequence may be a sequence located upstream (5' UTR) of the GRK1 promoter or PDE6B promoter found in the human genomic sequence or a portion thereof. More specifically, the UTR sequence may have a length of at least 1 bp, 2 bp, 3 bp, 4 bp, 5 bp, 6 bp, 7 bp, 8 bp, 9 bp, 10 bp, 20 bp, 30 bp, 40 bp, 50 bp, 60 bp, 70bp, 80 bp, 90 bp, 100 bp, 200 bp, 300 bp, 400 bp, 500 bp, 600 bp, 700 bp, 800 bp, 900 bp, 1 Kb, 2 Kb, 3 Kb, 4 Kb, 5 Kb, 6 Kb, 7 Kb, 8 Kb, 9 Kb or 10 Kb.

More preferably, the 5' UTR may be optimized for the desired protein expression level. The optimized sequence may have, for example, a nucleotide sequence of SEQ ID NO: 8 or SEQ ID NO: 9, but is not limited thereto. More specifically, the 5' UTR binding to the promoter of the present disclosure is preferably the UTR of the inserted gene.

The expression cassette according to the present disclosure may comprise a polyA sequence. The polyA sequence may be preferably a polyA sequence derived from the PCK6 AS1 gene (SEQ ID NO: 35) or a polyA sequence derived from the Bovine Growth Hormone (BGH) gene (SEQ ID NO: 36), and more preferably a polyA sequence derived from the BGH gene.

The expression cassette according to the present disclosure preferably comprises an operably linked enhancer. Specifically, any one enhancer selected from the group consisting of SEQ ID NOs: 12 to 18 and SEQ ID NOs: 28 to 31 is further included. Sequences showing functional homology to the sequences described above as SEQ ID NOs: 12 to 18 and showing at least 90% sequence identity are also included within the scope of the present disclosure.

More preferably, the present disclosure provides an expression cassette comprising: a. a promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7; b. any one enhancer selected from the group consisting of SEQ ID NOs: 12 to 18 and SEQ ID NOs: 28 to 31; and c. a nucleotide encoding a polypeptide operably linked thereto.

The enhancer sequence element may be present at any location, such as downstream or upstream of the gene. Preferably, the enhancer sequence element may be present upstream of the gene.

If desired, the expression cassette may further comprise a spacer sequence of any number of nucleotides from 1 to 1000. Such a spacer may be present between the enhancer and the promoter, and may be any sequence that does not affect the action of the respective sequences. According to an embodiment of the present disclosure, the spacer may comprise nucleotides of SEQ ID NO: 25 or SEQ ID NO: 34.

The enhancer according to the present disclosure is derived from a sequence at the region 2291 to 2440 of intron 4 of GRK1 and may be a nucleotide of SEQ ID NO: 12.

In addition, the sequence may be shortened further to include any one or more selected from the group consisting of SEQ ID NOs: 13 to 18. In addition, any sequence thereof may be repeated at least once, twice, or three times.

The expression cassette of the present disclosure may comprise a promoter, an enhancer, and one or more nucleic acids operably linked thereto. For example, the promoter and the enhancer may be operably linked to a nucleic acid encoding one or more therapeutic genes.

More preferably, the present disclosure provides an expression cassette comprising: a. a promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7; b. any one enhancer selected from the group consisting of SEQ ID NOs: 12 to 18 and SEQ ID NOs: 28 to 31; c. the 5'UTR of SEQ ID NO: 8 or SEQ ID NO: 9; and d. a nucleotide encoding a polypeptide operably linked thereto.

If desired, the expression cassette according to the present disclosure further comprises an RS1 promoter sequence or a sequence having at least 90% identity thereto. More specifically, the expression cassette may further comprise a promoter region of SEQ ID NO: 22 or a sequence having at least 90% identity thereto. It may be desirable for such a sequence to be located downstream of the enhancer based on the enhancer.

Preferably, the expression cassette according to the present disclosure may be an expression cassette comprising: a. a promoter of 100 to 400 nucleotides in length comprising as a core nucleotide sequence a nucleotide sequence having at least 90% identity to the sequence of SEQ ID NO: 7, or the sequence of SEQ ID NO: 7; b. an enhancer of SEQ ID NO: 12; and c. a nucleotide encoding a polypeptide operably linked thereto.

Preferably, the expression cassette according to the present disclosure may be an expression cassette comprising: a. a promoter of 100 to 400 nucleotides in length comprising as a core nucleotide sequence a nucleotide sequence having at least 90% identity to the sequence of SEQ ID NO: 7, or the sequence of SEQ ID NO: 7; b. an enhancer of SEQ ID NO: 12; c. the 5'UTR of SEQ ID NO: 9; and d. a nucleotide encoding a polypeptide operably linked thereto. More specifically, the 5' UTR binding to the promoter of the present disclosure is preferably the UTR of the inserted gene.

Preferably, the expression cassette according to the present disclosure may be an expression cassette comprising: a. a RS1 promoter of SEQ ID NO: 22 or a nucleotide sequence having at least 90% identity thereto; b. an enhancer of SEQ ID NO: 12; c. a promoter of 100 to 400 nucleotides in length comprising as a core nucleotide sequence a nucleotide sequence having at least 90% identity to the sequence of SEQ ID NO: 7, or the sequence of SEQ ID NO: 7; d. the 5'UTR of SEQ ID NO: 9; and e. a nucleotide encoding a polypeptide operably linked thereto. Here, the enhancer may be positioned between the RS1 promoter of SEQ ID NO: 22 and the PDE6B promoter sequence of SEQ ID NO: 7, and a spacer may be further included between these sequences. More specifically, the 5' UTR binding to the promoter of the present disclosure is preferably the UTR of the inserted gene.

Preferably, the expression cassette according to the present disclosure may be an expression cassette comprising: a. a promoter of 100 to 400 nucleotides in length comprising as a core nucleotide sequence a nucleotide sequence having at least 90% identity to the sequence of SEQ ID NO: 7, or the sequence of SEQ ID NO: 7; b. an enhancer of SEQ ID NOs: 30 to 31; c. the 5'UTR of SEQ ID NO: 24; and d. a nucleotide encoding a polypeptide operably linked thereto. More specifically, the 5' UTR binding to the promoter of the present disclosure is preferably the UTR of the RS1 gene (SEQ ID NO: 24).

The expression cassette may highly express nucleotides encoding a polypeptide operably linked to a photoreceptor specifically.

The present disclosure also provides a vector comprising the above-described promoter, and/or expression cassette.

The term "vector" refers to a nucleic acid molecule used as a vehicle for delivering genetic material, and particularly for delivering nucleic acids *in vitro* or *in vivo* into a host cell. Vectors include, but are not limited to, plasmids, phasmids, cosmids, transportable elements, viruses, and artificial chromosomes (e.g., YAC).

Preferably, the vectors of the present disclosure are suitable vectors for use in gene or cell therapy, and are particularly suitable for targeting retinal cells, more specifically photoreceptor cells.

The vector of the present disclosure is preferably a viral genomic vector comprising any element required to establish expression of the desired polypeptide in a host cell, such as, a promoter, for example, the promoter of the present disclosure, an ITR, a ribosome binding element, an enhancer, a selection marker, an intron, a polyA signal, and/or a replication origin.

In some embodiments, the vectors are viral vectors derived from viruses such as a Moloney murine leukemia virus vector (MoMLV), murine stem cell virus (MSCV), spleen focus-forming virus (SFFV), myeloproliferative sarcoma virus (MPSV) or SNV, a lentiviral vector (e.g., derived from human immunodeficiency virus (HIV), simian immunodeficiency virus (SIV), feline immunodeficiency virus (FIV), bovine immunodeficiency virus (BIV) or equine infectious anemia virus (EIAV)), an adenovirus (Ad) vector, an adeno-associated virus (AAV) vector, an anellovirus vector, a simian virus 40 (SV-40) vector, a bovine papilloma virus vector, an Epstein-Barr virus vector, a herpes virus vector, a vaccinia virus vector, a Harvey murine sarcoma virus vector, a murine mammary tumor virus vector, an Anellovirus vector, and a Rous sarcoma virus vector.

In a particular embodiment, the vector is a retroviral vector, preferably a lentiviral vector or a non-pathogenic parvovirus.

As commonly understood in the art, depending on the particular viral vector contemplated for use, suitable sequences such as AAV ITR for AAV vectors, or LTR for lentiviral vectors, may be introduced into the vectors of the present disclosure to obtain a functional viral vector.

In some embodiments, the vector is a non-viral vector. Non-viral vectors are largely divided into cationic polymers and cationic liposomes. Cationic polymers refer to polymers comprising artificially synthesized or naturally formed cations, and cationic lipid particles refer to artificially prepared phospholipid vesicles with bilayer membranes. Non-viral vectors include, for example, lipid nanoparticles (LNPs), polymer nanoparticles (PNPs), exosomes, and virus-like particles (VLPs), but are not limited thereto. The non-viral vector may be delivered into cells using any method known in the art, including calcium phosphate coprecipitation, liposome transfection, cell fusion, receptor-directed gene transfer, naked DNA injection, electroporation, bioballistic flight, or molecular acceleration.

In a preferred embodiment, the vector is an AAV vector.

Human parvovirus adeno-associated virus (AAV) is a naturally replication-deficient dependovirus capable of integrating into the genome of infected cells to establish a potential infection. The AAV vector is known to be a safe vector because most (>99%) of therapeutic genes delivered into the nucleus are not inserted into the cell gene but are expressed in a transient expression state for a long time. In the case of wild-type AAV (wtAAV; wild-type AAV), the Rep gene is triggered by a helper virus such as herpes (HSV; Herpes Simplex Virus), adenovirus (Ad; Adenovirus), etc., allowing replication both *in vivo* and *in vitro.* However, recombinant AAV (rAAV), engineered for therapeutic purposes, lacks the Rep gene and is utilized as a replication-deficient viral vector both *in vivo* and *in vitro.* In addition, it is known that the Rep gene of wtAAV may be inserted into a specific site in the human genome, called AAVS1, located on chromosome 19 in humans (19ql3.3-qter) with a low probability (<0.01%), but in the case of rAAV, the Rep gene was removed from this site, thereby ensuring safety. Thus, AAV has attracted great interest as a potential vector for human gene therapy. This virus has several advantageous properties, including the lack of association with any human diseases, the ability to infect both dividing and non-dividing cells, and the ability to infect a wide range of cell lines derived from a variety of tissues.

As used herein, the term "AAV vector" refers to a polynucleotide vector comprising at least one AAV inverted terminal repeat (ITR) sequence, and preferably two ITRs flanking one or more heterologous sequences (i.e., nucleic acid sequences not of AAV origin). Such AAV vectors, when present in a host cell, may be replicated and packaged into infectious viral vector particles expressing AAV rep and cap gene products, i.e., the AAV Rep and Cap proteins, that are infected with a suitable helper virus (or expressing a suitable helper function).

The term "inverted terminal repeat" or "ITR" sequence is a well-understood term in the art and refers to a relatively short sequence found at the end of the viral genome that exists in a contralateral orientation. The "AAV inverted terminal repeat (ITR)" sequence is approximately a 145-nucleotide sequence present on both ends of a native, single-stranded AAV genome. The outermost 125 nucleotides of the ITR exist in one of two alternating orientations, creating heterogeneity between different AAV genomes and between the two ends of a single AAV genome. The outermost 125 nucleotides also contain several shorter regions of self-complementarity, allowing for the in-chain base pairing that occurs within this site of the ITR. An AAV ITR for use in a vector of the present disclosure may have a wild-type nucleotide sequence or may be altered by insertion, deletion, or substitution. The serotype of the inverted terminal repeat (ITR) of the AAV vector may be selected from any known human or non-human AAV serotype.

When an AAV vector is introduced into a larger polynucleotide (e.g., intrachromosomal or other vectors such as plasmids used for cloning or transfection), the AAV vector may be referred to as a "pro-vector" that can be "rescued" by cloning and capsizing in the presence of AAV packaging functions and suitable helper functions. The AAV vector of the present disclosure may be in any of a number of forms, including, but not limited to, plasmids, linear artificial chromosomes complexed with lipids, encapsulated within liposomes, and encapsidated within viral particles, such as AAV particles.

The promoter or expression cassette of the present disclosure may be introduced into the vector by any method known to those skilled in the art.

The vector of the present disclosure may be packaged into a viral capsid that produces "viral particles". Accordingly, the present disclosure is also directed to viral particles comprising the vectors of the present disclosure.

In a particular embodiment, the vector is an AAV vector and is packaged into an AAV-derived capsid to produce an "adeno-associated virus particle" or "AAV particle". Thus, as used herein, the term "AAV particle" refers to a viral particle consisting of at least one AAV capsid protein and an encapsidated AAV vector genome.

The capsid serotype determines the tropism range of AAV particle.

A number of serotypes of adeno-associated virus (AAV), including 12 human serotypes and 100 or more serotypes from non-human primates, have now been identified. Other currently used AAV serotypes include, but are not limited to, AAV1, AAV3, AAV4, AAV5, AAV6, AAV7, AAV8, AAVrh8, AAV9, AAV10, AAVrh10, AAV11, AAV12, AAVrh74 and AAVdj. In addition, examples of the non-naturally processed variant may include AAV2.7m8, AAVLK03, AAV-Spark9001, AAV-R100, AAV-C102, AAV-A101, AAVr3.45, AAV-PHP.eB, AAV-PHP.S, AAV-retro, AAV-QuadYF, and preferably, AAV2.7m8, AAVLK03, AAV-Spark9001, AAV-R100, AAV-C102. In particular, the capsid protein may be a variant comprising one or more amino acid substitutions that improve transduction efficiency.

Different AAV serotypes are used to optimize transduction of specific target cells or target specific cell types within a particular target tissue (e.g., retinal cells). The AAV particles may comprise viral proteins and viral nucleic acids of the same serotype or any natural or artificial sequence variant of the AAV. For example, the AAV particle may comprise an AAV2 capsid protein and at least one, preferably two AAV2 ITRs. Any combination of AAV serotypes for the production of AAV particles is provided herein as each combination is represented and described herein.

AAV viruses may be processed using conventional molecular biology techniques, enabling these particles to be optimized for cell-specific delivery of nucleic acid sequences, reduced immunogenicity, adjusted stability and particle life, effective degradation, and precise delivery to the nucleus.

As an alternative to using AAV native serotypes, artificial AAV serotypes may be used in the context of the present disclosure, including, but not limited to, AAVs with capsid proteins that are non-naturally present. Such artificial capsids may be produced by any suitable technique using a selected AAV sequence (e.g., a fragment of a VP1 capsid protein) in combination with a heterologous sequence obtainable from a different, selected AAV serotype, that is a non-contiguous portion of the same AAV serotype either from a non-AAV virus source or from a non-viral source. The artificial AAV serotype may be, but is not limited to, a chimeric AAV capsid or a mutated AAV capsid.

The chimeric capsid comprises a VP capsid derived from a VP capsid protein derived from at least two different AAV serotypes or comprises at least one chimeric VP protein that binds a VP protein region or domain derived from at least two AAV serotypes.

The capsid protein may also be mutated, particularly to improve the transfection efficacy. The mutated AAV capsid may be obtained from capsid variants inserted by error prone PCR and/or peptide insertion or by incorporating one or more amino acid substitutions. In particular, the mutation may be made at any one or more of the tyrosine residues of a native or non-native capsid protein (e.g., VP1, VP2, or VP3). Preferably, the mutated residue is a surface exposed tyrosine residue. Exemplary mutations include, but are not limited to, tyrosine to phenylalanine substitutions such as Y252F, Y272F, Y444F, Y500F, Y700F, Y704F, Y730F, Y275F, Y281F, Y508F, Y576F, Y612G, Y673F and Y720F.

In a preferred embodiment, the AAV particles comprise AAV2-derived capsids. In this embodiment, the capsid may comprise one or more tyrosine to phenylalanine substitutions, preferably Y444F substitutions.

A number of methods are known in the art for the production of viral particles, and particularly AAV particles, including transfection, stable cell line production, and infectious hybrid virus production systems including adenovirus-AAV hybrids, herpesvirus-AAV hybrids, and baculovirus-AAV hybrids.

The necessary elements for the production of AAV virus particles,

in the case of animal cell line-based production, include: 1) an animal cell line such as HeLa, A549, CHO or HEK293 cells; 2) a helper plasmid encoding adenovirus genes E4, E2A, VA or a helper virus such as adenovirus (Ad) or herpesvirus (HSV); 3) AAV rep and cap genes and gene products; 4) at least one AAV ITR sequence, e.g., a nucleic acid encoding a base sequence of a therapeutic gene or reporter protein between the ITR sequences by the vector of the present disclosure; and 5) an AAV production-suitable medium and medium components optimized for adherent cell culture or suspension cell culture.

The necessary elements for the production of AAV virus particles, in the case of insect cell line-based production, include: 1) an insect cell line derived from SF9 or SF9; 2) a baculovirus or baculovirus-derived virus capable of introducing a genetic trait into SF9; 3) a Rep, Cap gene, ITR-therapeutic gene/reporter protein coding, E4, E2A, VA information of helper plasmids contained in the baculovirus or baculovirus-derived virus, and transducible by being divided into two or three or more vectors; and 4) AAV production-suitable medium and medium components optimized for SF9 or SF9-derived insect cell suspension culture.

More preferably, the present disclosure relates to an AAV particle comprising the vector according to the present disclosure and an AAV-derived capsid.

The present disclosure also relates to isolated host cells transformed or transfected with the expression cassette, the vector, or the viral particles of the present disclosure.

The host cell may be any animal cell, plant cell, bacterial cell, or yeast. Preferably, the host cell is a mammalian cell or an insect cell. More preferably, the host cell is a human cell.

In a preferred embodiment, the host cell is a human retinal cell.

The expression cassette or vector of the present disclosure may include, but are not limited to, calcium phosphate-DNA precipitation, DEAE-dextran transfection, electroporation, microinjection, biolistics, lipofection, or viral infection, and may be maintained in ectopic form in a host cell or integrated into the genome.

In a preferred embodiment, the expression cassette or vector of the present disclosure is transfected into a host cell, preferably using the viral particles of the present disclosure, more preferably using the AAV particles of the present disclosure.

The present disclosure also provides a composition for enhancing expression of a target gene in the retina, comprising the expression cassette, the vector, the viral particle, or the cell of the present disclosure. Preferably, the present disclosure provides a composition for enhancing expression of a target gene in photoreceptor cells, comprising the expression cassette, the vector, the virus particle, or the cell of the present disclosure.

The present disclosure also relates to a pharmaceutical composition comprising the expression cassette, the vector, the viral particle or the cell of the present disclosure.

Such compositions comprise a therapeutically effective amount of a therapeutic agent (an expression cassette, vector, viral particle or cell of the present disclosure), and a pharmaceutically acceptable excipient. As used herein, the term "pharmaceutically acceptable" means approved by a regulatory body, such as the European Pharmacopeia, or a recognized pharmacopeia, for use in animals and/or humans. The term "excipient" refers to a diluent, adjuvant, carrier, or vehicle with which a therapeutic agent is administered.

Pharmaceutically acceptable excipients, as well known in the art, are relatively inert substances that facilitate administration of the pharmacologically effective substance and may be supplied as a liquid solution or suspension, as an emulsion, or in a solid form suitable for dissolving or suspending in a liquid immediately prior to use. For example, the excipient may provide morphology or consistency, or may act as a diluent. Suitable excipients include, but are not limited to, stabilizers, wetting and emulsifying agents, salts to change osmotic pressure, encapsulants, pH buffering substances, and buffering agents. Such excipients include any pharmaceutical agents suitable for direct delivery to the eye that can be administered without excessive toxicity. Pharmaceutically acceptable excipients include, but are not limited to, sorbitol, any of the various tween compounds, and liquids such as water, saline, glycerol and ethanol. Pharmaceutically acceptable salts may include, for example, inorganic acid salts such as hydrochloride, hydrobromide, phosphate, sulfate, and the like; and salts of organic acids such as acetate, propionate, malonate, benzoate, and the like. A complete discussion of pharmaceutically acceptable excipients is available in Remington's Pharmaceutical Sciences, 15th Edition.

Preferably, the composition is formulated to be administered, in particular, by intraocular injection, e.g., subretinal and/or intravitreal administration. Thus, the composition may be combined with a pharmaceutically acceptable excipient, such as saline, Ringer's balanced salt solution (pH 7.4), and the like.

The pharmaceutical compositions described herein may be packaged in a single unit dose form or in multi-dose form.

In an embodiment, the pharmaceutical composition comprises a vector or viral particle of the present disclosure, more preferably an AAV vector or particle.

In another embodiment, the pharmaceutical composition comprises a host cell of the present disclosure, preferably a human host cell of the present disclosure transformed or transfected with an expression cassette, vector or viral particle, preferably an AAV particle, of the present disclosure.

Optionally the composition comprising the host cell may be frozen for storage at any temperature suitable for storage of the cell. For example, the cells may be frozen at about -20°C to -80°C or any other suitable temperature. Cryofrozen cells may be stored in an appropriate container and manufactured for storage, thereby reducing the risk of cell damage and maximizing the tendency of the cells to withstand thawing. In addition, the cells may be maintained at room temperature of the refrigeration, e.g., at about 4°C.

The amount of pharmaceutical composition to be administered may be measured by standard procedures well known to those skilled in the art.

Physiological data (e.g., age, physique, and body weight) of the patient and the type and severity of the disease to be treated should be considered to determine the appropriate dosage.

The pharmaceutical composition of the present disclosure may be administered in a single dose or multiple doses.

In a particular embodiment, the pharmaceutical composition comprises the viral particles of the present disclosure and each unit dose comprises between 10⁸ and 10¹² viral particles, preferably between 10⁹ and 10¹² viral particles.

The pharmaceutical composition may also comprise one or several additional active ingredients, such as corticosteroid, antibiotic, analgesic, immunosuppressive agent, trophic factor, or any combination thereof.

The present disclosure provides a pharmaceutical composition for preventing or treating an eye disease, comprising the expression cassette according to the present disclosure or a vector including the expression cassette.

In a further aspect, the present disclosure also provides
- a pharmaceutical composition of the present disclosure for use in the treatment of an eye disease,
- an expression cassette, vector, viral particle or host cell of the present disclosure for use in the treatment of an eye disease,
- the use of an expression cassette, vector, viral particle or host cell of the present disclosure for the manufacture of a medicament for the treatment of an eye disease, and
- a method of treating an eye disease comprising administering a therapeutically effective amount of the pharmaceutical composition of the present disclosure to a subject in need thereof.

The present disclosure relates to a method for treating an eye disease, comprising administering an expression cassette or a vector comprising the same to a subject in need thereof.

There is also provided a method for enhancing expression of a target gene in the retina, comprising administering the expression cassette or the vector comprising the same to a subject in need thereof. Preferably, the present disclosure provides a method for enhancing expression of a target gene in a photoreceptor cell, comprising administering the expression cassette or the vector comprising the same to a subject in need thereof.

There is also provided a method for enhancing expression of a target gene in retinal cells, comprising treating retinal cells with the expression cassette or the vector comprising the same in Ex vivo and/or *in vitro.* Preferably, the retinal cells are photoreceptor cells.

In an embodiment, the eye disease is a retinal disease.

Examples of retinal diseases include, but are not limited to, retinitis pigmentosa (RP), including X-linked, recessive, dominant and sporadic forms, rod-cone dystrophy, Usher's syndrome, Stargardt's disease, cone-rod dyschromatopsia, cone dyschromatopsia, complete achromatopsia, blue cone monochromacy, enhanced S-cone syndrome, rod dyschromatopsia, choroideremia, Leber's congenital amaurosis, juvenile X-chromosome linked retinoschisis (JXLR), fundus albipunctatus, retinitis punctata albescens, fleck retina of Kandori, bietti crystalline retinal dystrophy, fenestrated sheen macular dystrophy, adult-onset foveomacular vitelliform dystrophy, Batten's disease, congenital stationary night blindness, familial exudative vitreoretinopathy (FEVR), ocular albinism, oculocutaneous albinism, fovea hypoplasia, abetalipoproteinemia, Stickler syndrome, retinal dystrophy (Bothnia type), crystalline maculopathy (associated with drugs or hyperoxaluria), cystinosis, Sjogren-Larsson syndrome, west African crystalline maculopathy, solar retinopathy, talc retinopathy, diabetic retinopathy, sickle cell retinopathy, macular telangectasia, eales disease, peripheral retinoschisis, central/branch retinal artery occlusion (CRAO/BRAO), central/branch retinal vein occlusion (CRVO/BRVO), haemorrhagic occlusive retinal vasculitis (HORV), drug-induced maculopathy, including chloroquine, hydroxychloroquine, phenothiazine, quinine sulfate, thioridazine, clofazimine, chlorpromazine, deferoxamine, chloroquine derivatives, cisplatin, carmustine, clofazimine, and vigabatrin; crystal-induced maculopathy, including tamoxifen, talc, canthaxanthin, methoxyflurane, and nitrofurantoin; cystoid macular edema (CME), including epinephrine, latanoprost, and nicotinic acid; progressive outer retinal necrosis (PORN), acute retinal necrosis (ARN), CMV retinitis, sarcoidosis, acute syphilitic posterior placoid chorioretinitis, tuberculous chorioretinitis, toxoplasmic retinochoroiditis, posterior uveitis and retinal vasculitis, intermediate uveitis, pars planitis ± cystoid macular edema (CME), endophthalmitis (anterior and/or posterior), posterior scleritis, masquerade syndrome, multifocal choroiditis and panuveitis (MCP), punctate inner choroidopathy (PIC), birdshot retinochoroidopathy, retinitis pigmentosa, white dot retinopathy, acute macular neuroretinopathy (AMN), and acute zonal occult outer retinopathy (AZOOR).

As used herein, the term "treatment", "treat" or "treating" refers to any action intended to alleviate a patient's health status, such as therapy, prevention, prophylaxis, and delay of the onset of disease. In some embodiments, this term refers to the alleviation or eradication of a disease or condition associated with the disease. In other embodiments, the term refers to minimizing the spread or exacerbation of a disease resulting from administering one or more therapeutic agents to a subject affected by the disease.

In particular, the term "treatment of eye disease" may refer to a treatment for providing improved vision, preventing the progression of the disease to full blindness, preventing the spread of damage to uninjured eye cells, improving damage to injured eye cells, preventing the occurrence of retinal damage or rescuing eyes with mild or advanced diseases. In some embodiments, the term refers to preventing, reducing, or stopping retinal cell degeneration by providing a therapeutic protein that corrects a genetic defect in a patient.

A "therapeutically effective amount" is intended to be an amount of a pharmaceutical composition of the present disclosure administered to a subject sufficient to constitute a treatment for an eye disease as defined above.

In the method of the present disclosure, the expression cassette, vector and/or pharmaceutical composition of the present disclosure is preferably administered intraocularly, and more preferably via subretinal or intravitreal administration.

The method of the present disclosure may also comprise administering at least one additional therapeutic agent to the subject. In particular, the therapeutic agent may be selected from the group consisting of corticosteroids, antibiotics, analgesics, immunosuppressants, or nutritional factors, or any combination thereof.

The composition of the present disclosure may be administered before or after the disease has symptoms, such as before or after partial or complete retinal cell degeneration and/or before or after partial or complete loss of vision.

The present disclosure also relates to a non-human animal model comprising the expression cassette, vector, viral particle or host cell of the present disclosure.

These animal models may be used for *in vivo* studies of retinal cell function. When using the promoters, cassettes, vectors or viral particles of the present disclosure, it is possible to identify or track retinal cells or monitor their activity, for example, through the expression of reporter proteins or voltage- or calcium-sensitive proteins.

The non-human animal model may also be used in screening methods to identify or select pharmaceutical agents acting on retinal cells.

Preferably, the non-human animal model is mammalian, more preferably primate, rodent, rabbit or mini-pig. The promoter, expression cassette or vector may be maintained in an episomal form in the cells of the model or integrated into the genome thereof.

Methods for transfecting or transforming animal cells, or producing transgenic animals that express a desired nucleic acid sequence under the control of a selected promoter-specifically, the promoter of the present disclosure-are well known to those skilled in the art and may be readily applied depending on the type of cell and animal.

All characteristics set forth herein (including all attached claims, abstract, and drawings) and/or all steps of any method or process so disclosed may be combined with any of the above embodiments in any combination, except for combinations in which at least some of these characteristics and/or steps are mutually exclusive.

All patents, patent applications, provisional applications, and publications described or cited herein are incorporated by reference in their entirety, including all drawings and Tables, to the extent they are not inconsistent with the apparent teachings herein.

The following embodiments are provided for illustrative purposes and are not intended to be limiting.

### [Advantageous Effects]

The promoter for gene expression in retinal cells and the vector system including the same according to the present disclosure may have a minimal unit for the action as a promoter, and unlike existing known promoters in retinal cells, may maintain a high expression level of the target gene, and thus can be utilized to maximize the expression of the target protein in retinal cells.

### [Description of Drawings]

For a better understanding of the present disclosure, and to illustrate how embodiments thereof may be implemented in practice, reference is now made to the accompanying drawings, provided by way of example:

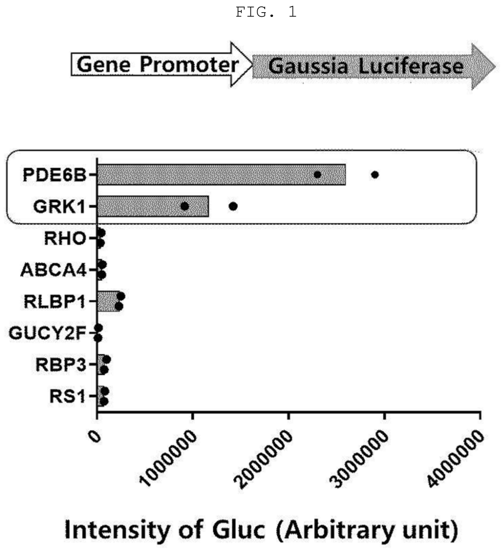

FIG. 1 shows the Gaussia Luciferase intensities of a promoter candidate group of expression cassettes targeted for overexpression in retinal cells.

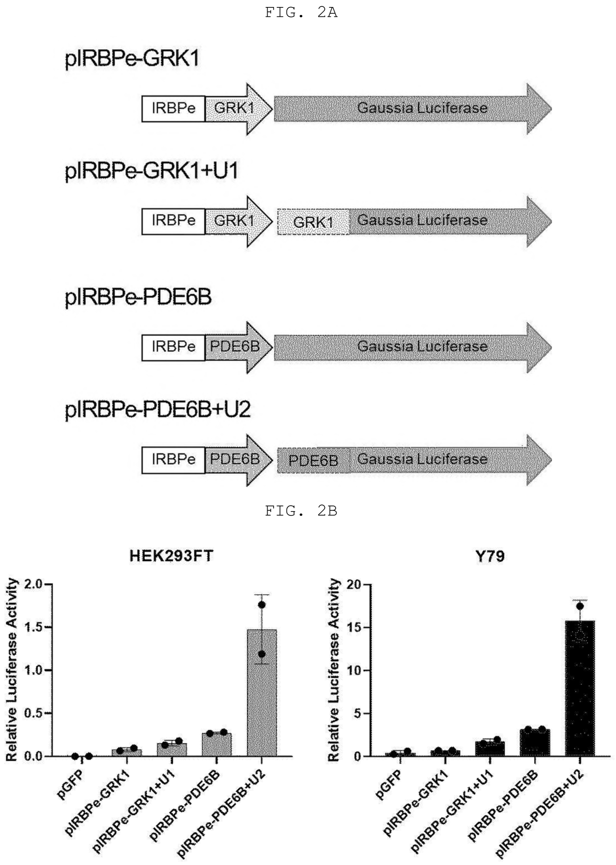

FIG. 2A shows designs of expression cassettes comprising a GRK1 promoter and a PDE6B promoter selected from the promoter candidate group and FIG. 2B shows the Gaussia Luciferase intensities thereof confirmed in HEK293FT and Y79 cells. The enhancer was IRBPe.

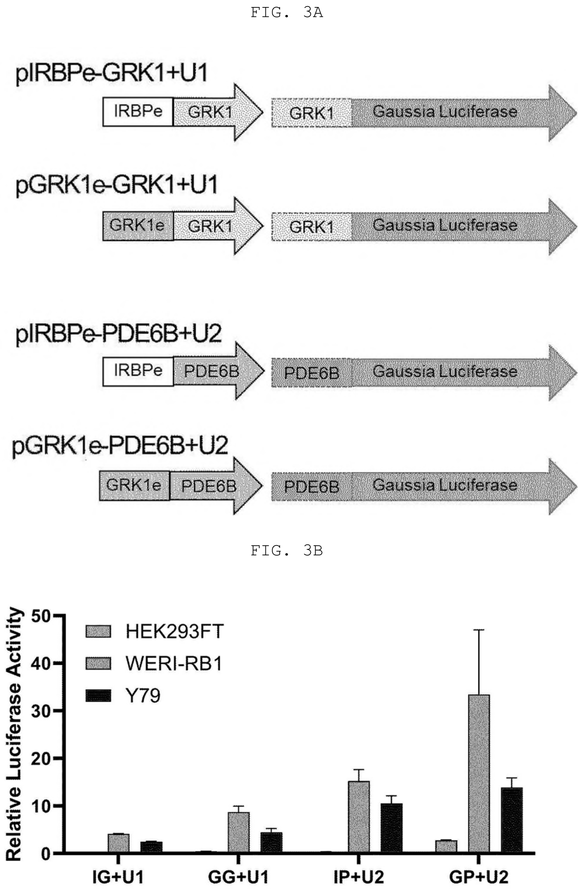

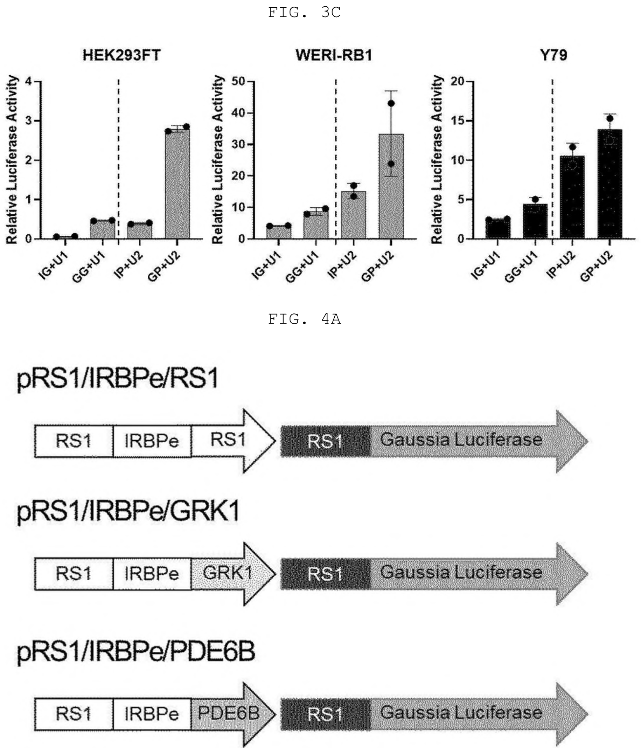

FIG. 3A shows expression cassettes designed by selecting an GRK1 IRBP-derived enhancer sequence (GRK1e) and an IRBP enhancer (IRBPe) as new enhancer candidate group, and binding them to the GRK1 promoter and the PDE6B promoter selected in FIG. 2, and FIGS. 3B and 3C show the Gaussia Luciferase intensities thereof confirmed in HEK293FT, WERI-RB1 and Y79 cells.

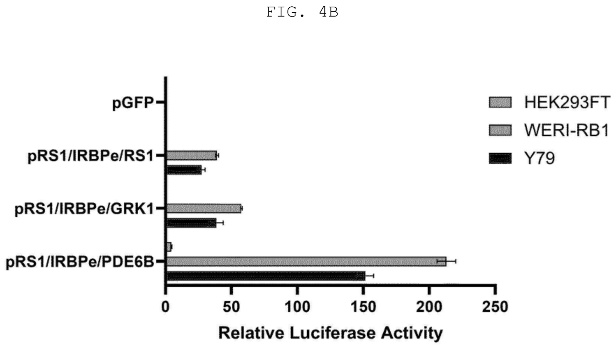

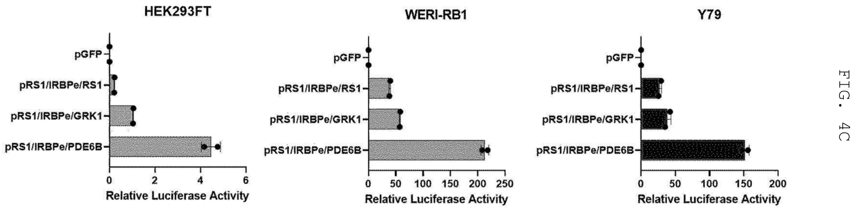

FIG. 4A shows the GRK1 promoter and PDE6B promoter simply inserted into a known photoreceptor-specific expression cassette (RS1/IRBPe/RS1) and FIGS. 4B and 4C show changes in relative tissue-specific expression efficiency confirmed in HEK293FT, WERI-RB1 and Y79 cells.

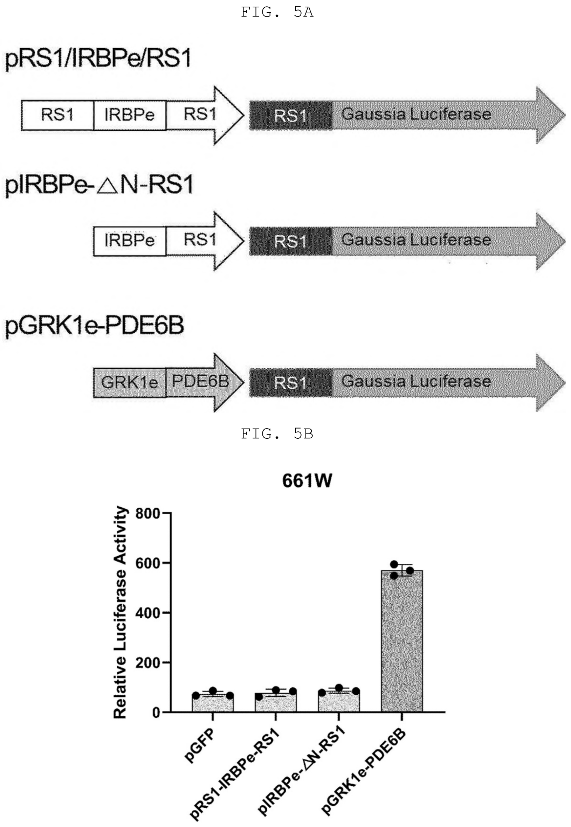

FIG. 5A shows removal of the N-terminus from a known photoreceptor-specific expression cassette (RS1/IRBPe/RS1) and insertion of the GRK1e-PDE6B cassette selected in FIG. 3, and FIG. 5B shows expression effect of the expression cassette system in photoreceptor cells 661W.

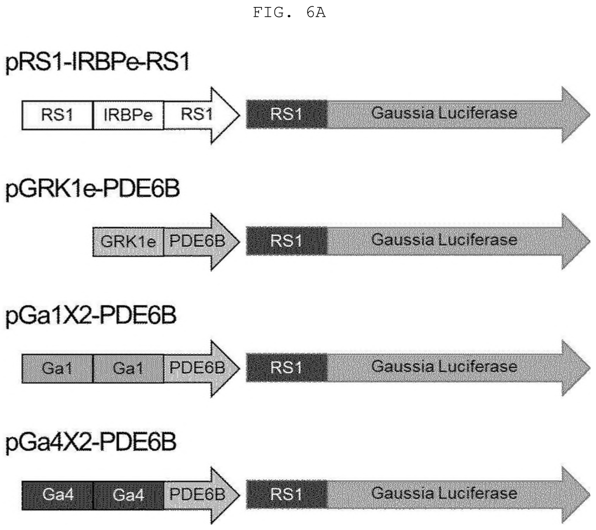

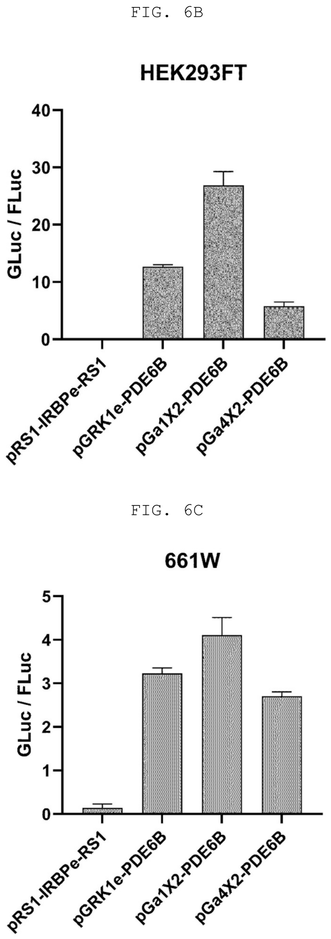

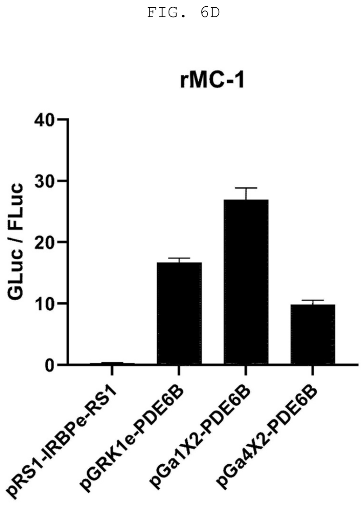

FIG. 6A shows vectors designed to confirm the effect of increasing and decreasing expression according to the repetition of some sequences in the GRK1-derived enhancer (GRK1e) sequence, FIG. 6B shows the expression effect in HEK293FT, FIG. 6C shows the expression effect in 661W, and FIG. 6D shows the expression effect in rMC1 cells.

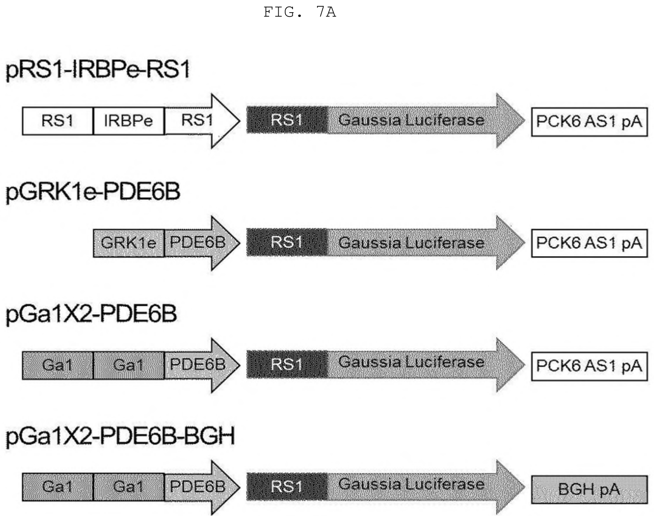

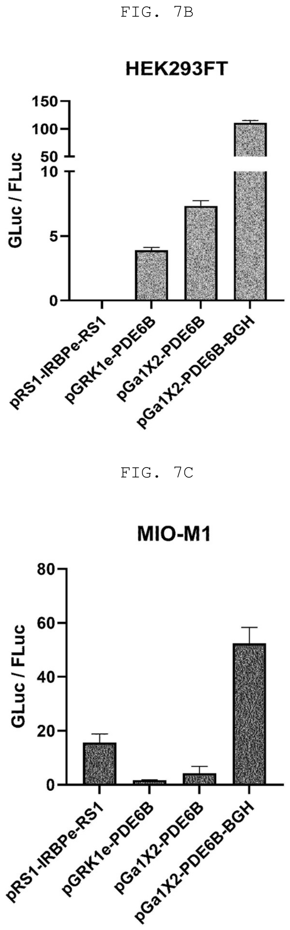

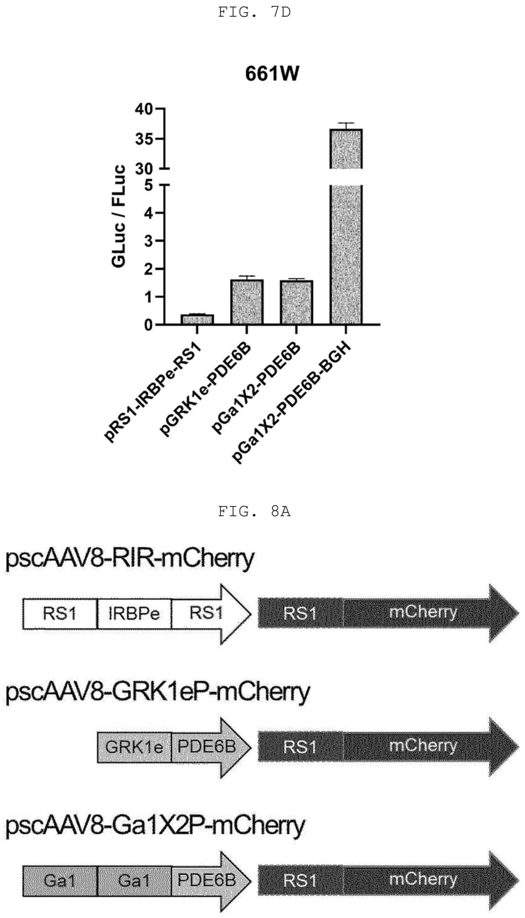

FIG. 7A shows the design of vectors in which the polyA signal sequence was replaced with the Bovine Growth Hormone gene-derived sequence in the expression system of FIG. 6, FIG. 7B shows the expression effects in HEK293FT, FIG. 7C shows the expression effects in MIO-M1, and FIG. 7D shows the expression effects in 661W cells.

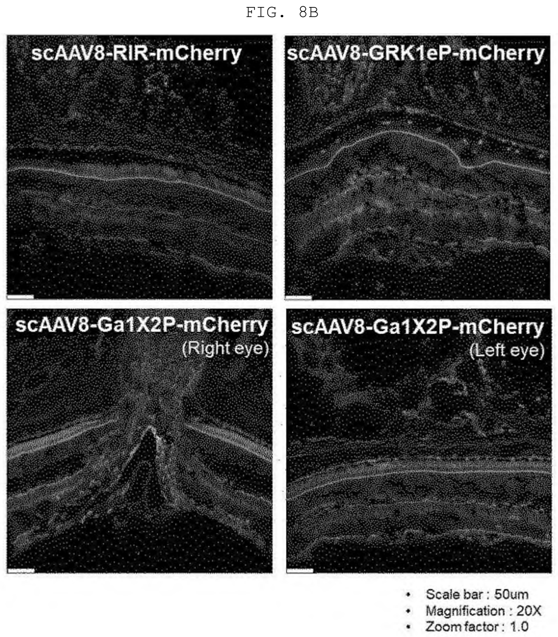

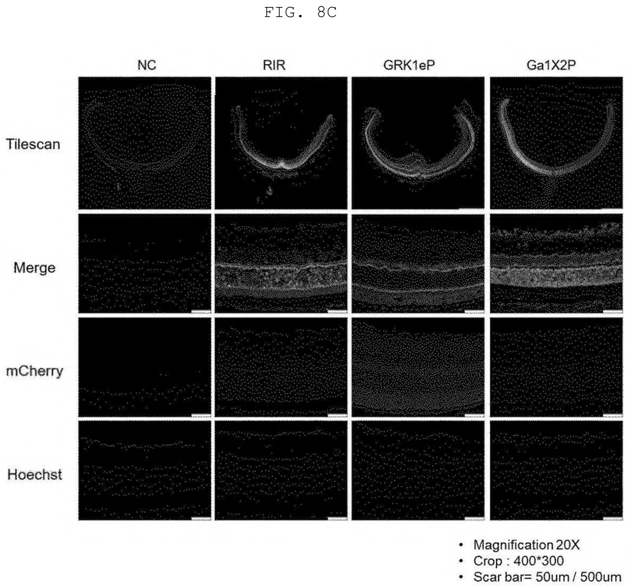

FIG. 8A shows the packaging of the expression cassette selected in FIG. 6 into adeno-associated virus serotype 8 to confirm the expression effect in the retina of a wild-type mouse, FIG. 8B shows the immunofluorescence staining results confirmed by intravitreal injection, and FIG. 8C shows the immunofluorescence staining results confirmed by subretinal injection.

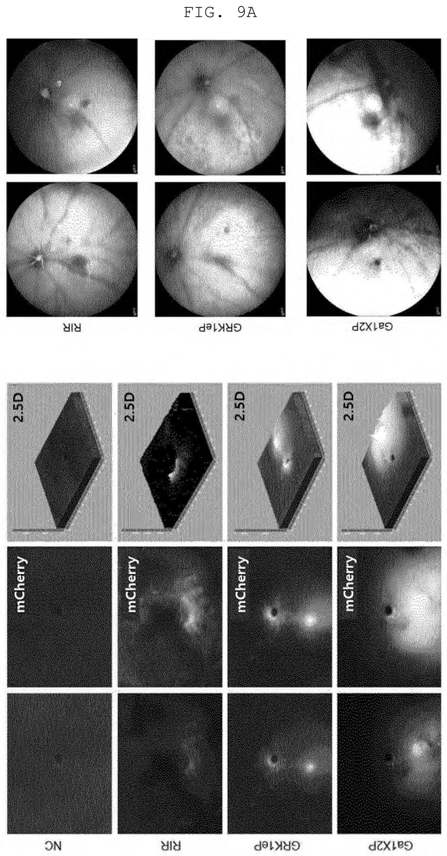

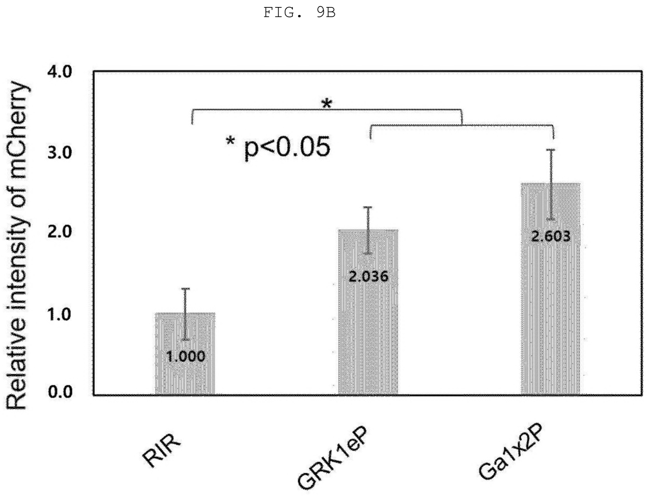

FIG. 9 shows the packaging of the expression cassette system according to the present disclosure into AAV serotype 8 in the sample as shown in FIG. 8 to confirm the expression effect in a wild-type mouse, FIG. 9A shows a fundus image obtained using scanning laser ophthalmoscopy (SLO), and FIG. 9B is a graph showing the quantification of the fundus image.

### [Best Mode]

Hereinafter, the present disclosure will be described in more detail through Examples. However, these Examples are provided to illustrate the present disclosure by way of example only, and the scope of the present disclosure is not limited to these Examples.

### Example 1. Culture and Maintenance of Cells

HEK293FT (R70007, ThermoFisher), Y79 (HTB-18, ATCC), WERI-RB1 (HTB-169-ATCC), rMC-1 (ENW001, Kerafast), MIO-M1 (RPID: CVCL_0433, received from Prof. G. Astrid Limb, Institute of Ophthalmology, London, UK), and 661W (received from Prof. Muayyad Al-Ubaidi, University of Houston) were inoculated and cultured in a 100φ flask dish at a temperature of 37°C and a carbon dioxide concentration of 5%. Subculture was performed by inoculating at a 1:5 ratio every 2 to 3 days. HEK293FT, rMC-1, and 661W were cultured using DMEM and high glucose (Gibco, 11995-065), Y79 and WERI-RB1 were cultured using RPMI 1640 (Gibco, 31800-022), and MIO-M1 was cultured in DMEM, high glucose (Gibco, 11995-065) supplemented with 1% Glutamax (Gibco, 35050061). 10% FBS (Gibco, 12483020) and 1% PS (penicillin/streptomycin, Gibco, 15140122) were added to all media used.

### Example 2. Selection of Promoter Types for Introduction of New Promoter Sequences

In order to select retinal-specific promoters, genes specifically expressed in retinal tissue were examined. To this end, the type of promoter capable of operating as a retinal-specific promoter was selected. More specifically, PDE6B, GRK1, RHO, ABCA4, RLBP1, GUCY2F, RBP3, and RS1 promoters were selected as candidates, and a group of promoters that could potentially lead to high expression levels were selected by confirming Gaussia luciferase.

More specifically, the coding sequence of Gaussia Luciferase and the PCK6 AS1 poly A signal sequence were inserted into the 3' end of the above-described promoters. Then, the constructed vector was transferred to HEK293FT cells using 500 ng of Lipofectamine 2000 (11668019, Invitrogen). As a control, the vector 500 ng expressing Firefly luciferase by SV40 Promoter was delivered simultaneously. After 24 hours, the intensity of each Luciferase was quantified, and the Gaussia Luciferase intensity was divided into the intensity of Firefly Luciferase to confirm the expression change.

The results are shown in FIG. 1, and the promoters capable of greatly increasing the expression level were identified as PDE6B and GRK1.

In addition, the sequences of the PDE6B and GRK1 genes are relatively accurately identified, and the sequences of the corresponding promoters are known to include some 5'-UTR sequences. Considering this, the subsequent experiment was carried out using PDE6B and GRK1, which have high potential for development as promoters.

### Example 3. Progress of Promoter Optimization

In order to optimize the function and length of the promoter, various versions of the promoter were created. A promoter design was performed based on promoter sequences including or not including the 5'-UTR of GRK1 or PDE6B. At the 3' end of the promoter, the coding sequence of Gaussia Luciferase and the PCK6 AS1 poly A signal sequence were inserted.

Specifically designed sequence-related information is shown in Table 1 and FIG. 2A.

**[Table 1]**

| | **Enhancer** | **Promoter** | **5'UTR** |
|---|---|---|---|
| pIRBPe-GRK1 (SEQ ID NO: 1) | IRBPe (SEQ ID NO: 5) | GRK1 (SEQ ID NO: 6) | - |
| pIRBPe-GRK1+U1 (SEQ ID NO: 2) | IRBPe (SEQ ID NO: 5) | GRK1 (SEQ ID NO: 6) | 5'UTR (U1, SEQ ID NO: 8) |
| pIRBPe-PDE6B (SEQ ID NO: 3) | IRBPe (SEQ ID NO: 5) | PDE6B (SEQ ID NO: 7) | - |
| pIRBPe-PDE6B+U2 (SEQ ID NO: 4) | IRBPe (SEQ ID NO: 5) | PDE6B (SEQ ID NO: 7) | 5'UTR (U2, SEQ ID NO: 9) |

| | | | |
|---|---|---|---|
| ** As a spacer between the enhancer and the promoter sequence, "gtcgac" (SEQ ID NO: 25) was used.* ** The coding sequence of Gaussia Luciferase, which is linked downstream of the promoter end or 5'UTR, was as follows:* "atgggagtcaaagttctgtttgccctgatctgcatcgctgtggccgaggccaagccca ccgagaacaacgaagacttcaacatcgtggccgtggccagcaacttcgcgaccacggat ctcgatgctgaccgcgggaagttgcccggcaagaagctgccgctggaggtgctcaaaga gctggaagccaatgcccggaaagctggctgcaccaggggctgtctgatctgcctgtccc acatcaagtgcacgcccaagatgaagaagttcatcccaggacgctgccacacctacgaa ggcgacaaagagtccgcacagggcggcataggcgaggcgatcgtcgacattcctgagat tcctgggttcaaggacttggagcccctggagcagttcatcgcacaggtcgatctgtgtg tggactgcacaactggctgcctcaaagggcttgccaacgtgcagtgttctgacctgctc aagaagtggctgccgcaacgctgtgcgacctttgccagcaagatccagggccaggtgga caagatcaagggggccggtggtgactaa"(SEQ ID NO: 41). | | | |

As noted above, sequences of expression cassettes to be used for AAV were designed to include enhancers, promoters and 5'UTR sequences. In particular, in the present experiment, the known enhancer IRBP, was used to confirm the characteristics of the promoter.

The constructed vectors were transferred to HEK293FT and Y79 cells using 500 ng of Lipofectamine 2000. As a control, the vector 500 ng expressing Firefly luciferase by SV40 Promoter was delivered simultaneously. After 24 hours, the intensity of each Luciferase was quantified, and the Gaussia Luciferase intensity was divided into the intensity of Firefly Luciferase to confirm the expression change.

The results are shown in FIG. 2B, and both the GRK1 and PDE6B promoters according to the present disclosure were confirmed to exhibit expression enhancing efficiency above a certain level in HEK293FT Y79 cells. In particular, it was confirmed that the PDE6B promoter had superior efficacy.

In addition, it was confirmed that the expression level was improved in all cases including the 5'UTR sequence, and thus the sequence design was performed by including the 5'UTR sequence together in the promoter sequence.

### Example 4. Progress of Enhancer Optimization

To maximize the desired therapeutic efficacy, a high level of expression in retinal cells, etc., is required, and therefore, the enhancer was modified to further optimize the enhancement of the expression level.

Specifically, the experiment was performed by designing an expression cassette in a manner similar to Example 3 above, but inserting the GRK1 enhancer instead of the IRBP enhancer. The specific vector design thereof is shown in Table 2 and FIG. 3A.

**[Table 2]**

| | **Enhancer** | **Promoter** | **5'UTR** |
|---|---|---|---|
| pIRBPe-GRK1+U1 (SEQ ID NO: 2) | IRBPe (SEQ ID NO: 5) | GRK1 (SEQ ID NO: 6) | 5'UTR (U1, SEQ ID NO: 8) |
| pGRK1e-GRK1+U1 (SEQ ID NO: 10) | GRK1e (SEQ ID NO: 12) | GRK1 (SEQ ID NO: 6) | 5'UTR (U1, SEQ ID NO: 8) |
| pIRBPe-PDE6B+U2 (SEQ ID NO: 4) | IRBPe (SEQ ID NO: 5) | PDE6B (SEQ ID NO: 7) | 5'UTR (U2, SEQ ID NO: |
| | | | 9) |
| pGRK1e-PDE6B+U2 (SEQ ID NO: 11) | GRK1e (SEQ ID NO: 12) | PDE6B (SEQ ID NO: 7) | 5'UTR (U2, SEQ ID NO: 9) |

| | | | |
|---|---|---|---|
| ** As a spacer between the enhancer and promoter sequence, "gtcgac" (SEQ ID NO: 25) was used for the vectors of SEQ ID NO: 2 and SEQ ID NO: 4, and "aagaggtcgac" (SEQ ID NO: 34) was used for the vectors of SEQ ID NO: 10 and SEQ ID NO: 11.* ** The coding sequence of Gaussia Luciferase, which is linked downstream of 5'UTR, was as follows:* "atgggagtcaaagttctgtttgccctgatctgcatcgctgtggccgaggccaagccca ccgagaacaacgaagacttcaacatcgtggccgtggccagcaacttcgcgaccacggat ctcgatgctgaccgcgggaagttgcccggcaagaagctgccgctggaggtgctcaaaga gctggaagccaatgcccggaaagctggctgcaccaggggctgtctgatctgcctgtccc acatcaagtgcacgcccaagatgaagaagttcatcccaggacgctgccacacctacgaa ggcgacaaagagtccgcacagggcggcataggcgaggcgatcgtcgacattcctgagat tcctgggttcaaggacttggagcccctggagcagttcatcgcacaggtcgatctgtgtg tggactgcacaactggctgcctcaaagggcttgccaacgtgcagtgttctgacctgctc aagaagtggctgccgcaacgctgtgcgacctttgccagcaagatccagggccaggtgga caagatcaagggggccggtggtgactaa"(SEQ ID NO: 41). | | | |

An experiment was performed to confirm changes in the expression of Gaussia Luciferase in HEK293FT, Y79 and WERI-RB1 cells according to the method described in Example 3.

The results are shown in FIG. 3B and 3C, and it was confirmed that substitution of the commonly used IRBP enhancer with the GRK1 enhancer (SEQ ID NO: 12) resulted in a remarkably increased expression level.

Accordingly, a subsequent experiment was performed using the GRK1 enhancer as a main enhancer.

In addition, in order to optimize the GRK1 enhancer, sequences included therein, i.e., SEQ ID NOs: 13 to 18 and SEQ ID NOs: 28 to 31, were separately designed, and novel expression cassettes were constructed by replacing only the enhancer and combining it with the above-described promoters in the same manner.

### Example 5. Preparation of Combinatorial Expression Cassette of RS1 Promoter Region for Improving Tissue Specificity

The AAV8-scRS1/IRBP-hRS1 vector is a vector system prepared during clinical trials registered with NCT02317887, and is known as a vector for photoreceptor specific expression. However, the vector system has problems such as requiring high-dose administration, which may cause inflammatory reactions, or not showing sufficient efficacy when administered at low doses.

Accordingly, an expression cassette with improved expression was designed by using the GRK1 promoter and the PDE6B promoter designed to exhibit overexpression characteristics. Specifically, based on the sequence of scRS1/IRBP-hRS1, the sequence corresponding to hRS1 was changed to GRK1 and PDE6B promoters, and the efficacy thereof was examined.

More specifically, the expression cassette was designed in a manner similar to that of Example 3, and the results are shown in Table 3 and FIG. 4A.

**[Table 3]**

| | **Promoter** | **Enhancer** | **Promoter** | **5'UTR** |
|---|---|---|---|---|
| pRS1/IRBPe/RS1 (SEQ ID NO: 19) | RS1 (SEQ ID NO: 22) | IRBPe (SEQ ID NO: 5) | RS1 (SEQ ID 23) | NO: 5'UTR (SEQ ID NO: 24) |
| pRS1/IRBPe/GRK1 (SEQ ID NO: 20) | RS1 (SEQ ID NO: 22) | IRBPe (SEQ ID NO: 5) | GRK1 (SEQ ID 6) | NO: 5'UTR (SEQ ID NO: 24) |
| pRS1/IRBPe/PDE6 B (SEQ ID NO: 21) | RS1 (SEQ ID NO: 22) | IRBPe (SEQ ID NO: 5) | PDE6B (SEQ ID 7) | NO: 5'UTR (SEQ ID NO: 24) |

| | | | | |
|---|---|---|---|---|
| ** As a spacer between the enhancer and the promoter sequence, "gtcgac" (SEQ ID NO: 25) was used.* * *The coding sequence of Gaussia Luciferase, which is linked downstream of 5'UTR, was as follows:* “atgggagtcaaagttctgtttgccctgatctgcatcgctgtggccgaggccaagccca ccgagaacaacgaagacttcaacatcgtggccgtggccagcaacttcgcgaccacggat ctcgatgctgaccgcgggaagttgcccggcaagaagctgccgctggaggtgctcaaaga gctggaagccaatgcccggaaagctggctgcaccaggggctgtctgatctgcctgtccc acatcaagtgcacgcccaagatgaagaagttcatcccaggacgctgccacacctacgaa ggcgacaaagagtccgcacagggcggcataggcgaggcgatcgtcgacattcctgagat tcctgggttcaaggacttggagcccctggagcagttcatcgcacaggtcgatctgtgtg tggactgcacaactggctgcctcaaagggcttgccaacgtgcagtgttctgacctgctc aagaagtggctgccgcaacgctgtgcgacctttgccagcaagatccagggccaggtgga caagatcaagggggccggtggtgactaa”(SEQ ID NO: 41). | | | | |

An experiment was performed to confirm changes in expression in HEK293FT, Y79 and WERI-RB1 cells according to the method described in Example 3. The results are shown in FIG. 4B and FIG. 4C, and it was confirmed that modification to the PDE6B or GRK1 promoter resulted in improved activity compared to the expression cassette known as scRS1/IRBP-hRS1.

In addition, as the overall expression level increased, the expression in HEK293FT cells also partially increased, but the increase in expression in WERI-RB1 and Y79 cells was significantly higher in terms of specificity.

### Example 6: Confirmation of Retinal Cell-Specific Promoter Activity

The results enabled the selection of promoter and enhancer candidates capable of increasing an absolute expression level.

Under this background, a combination of the GRK1 enhancer and the PDE6B promoter was considered as a combination capable of greatly increasing retinal cell-specific expression, and the extent to which these combinations improved tissue specificity compared to the known scRS1/IRBP-hRS1 expression cassette system was evaluated.

For the comparison, the expression cassette was designed in a similar manner to Example 3, and the vector design is shown in Table 4 and FIG. 5A.

**[Table 4]**

| | **Promoter** | **Enhancer** | **Promoter** | **5'UTR** |
|---|---|---|---|---|
| pRS1/IRBPe/RS1 (SEQ ID NO: 19) | RS1 (SEQ ID NO: 22) | IRBPe (SEQ ID NO: 5) | RS1 (SEQ ID NO: 23) | 5'UTR (SEQ ID NO: 24) |
| pIRBPe-△N-RS1 (SEQ ID NO: 26) | - | IRBPe (SEQ ID NO: 5) | RS1 (SEQ ID NO: 23) | 5'UTR (SEQ ID NO: 24) |
| pGRK1e-PDE6B (SEQ ID NO: 27) | - | GRK1e (SEQ ID NO: 12) | PDE6B (SEQ ID NO: 7) | 5'UTR (SEQ ID NO: 24) |

| | | | | |
|---|---|---|---|---|
| ** As a spacer between the enhancer and promoter sequence, "gtcgac" (SEQ ID NO: 25) was used for the vectors of SEQ ID NO: 19 and SEQ ID NO: 26, and "aagaggtcgac" (SEQ ID NO: 34) was used for the vector of SEQ ID NO: 27.* ** The coding sequence of Gaussia Luciferase, which is linked downstream of 5'UTR, was as follows:* "atgggagtcaaagttctgtttgccctgatctgcatcgctgtggccgaggccaagccca ccgagaacaacgaagacttcaacatcgtggccgtggccagcaacttcgcgaccacggat ctcgatgctgaccgcgggaagttgcccggcaagaagctgccgctggaggtgctcaaaga gctggaagccaatgcccggaaagctggctgcaccaggggctgtctgatctgcctgtccc acatcaagtgcacgcccaagatgaagaagttcatcccaggacgctgccacacctacgaa ggcgacaaagagtccgcacagggcggcataggcgaggcgatcgtcgacattcctgagat tcctgggttcaaggacttggagcccctggagcagttcatcgcacaggtcgatctgtgtg tggactgcacaactggctgcctcaaagggcttgccaacgtgcagtgttctgacctgctc aagaagtggctgccgcaacgctgtgcgacctttgccagcaagatccagggccaggtgga caagatcaagggggccggtggtgactaa"(SEQ ID NO: 41). | | | | |

An experiment was performed to confirm a change in expression in 661W cells, which are photoreceptor cells, according to the method described in Example 3, and the result is shown in FIG. 5B.

As can be seen in FIG. 5B, both pRS1-IRBPe-RS1 and pIRBPe-△N-RS1 did not show tissue-specific expression efficacy in 661W cells, retinal photoreceptor cells. On the other hand, it was confirmed that pGRK1e-PDE6B exhibited a significantly improved expression effect specifically to photoreceptors in 661W cells.

In particular, these results show that the pGRK1e-PDE6B-based expression cassette according to the present disclosure is capable of greatly enhancing the expression of a target gene specifically to a target cell without the RS1 promoter, which is known to be photoreceptor cell-specific.

This confirms that the promoter and enhancer candidates according to the present disclosure may be utilized as a suitable expression cassette system compared to existing photoreceptor-specific expression systems.

### Example 7: GRK1 Enhancer Engineering and Confirmation of Expression Efficiency

Sequences (SEQ ID NOs: 30, 31) obtained by twice-repeating the Ga1 (SEQ ID NO: 28) or Ga4 (SEQ ID NO: 29) sequence derived from the GRK1 enhancer (SEQ ID NO: 17) were combined with the PDE6B promoter (SEQ ID NO: 7) and the RS1 5'UTR (SEQ ID NO: 24), respectively, to prepare novel expression cassettes (SEQ ID NOs: 32, 33).

Specific vector designs are shown in Table 5 and FIG. 6A.

**[Table 5]**

| | **Promoter** | **Enhancer** | **Promoter** | **5'UTR** |
|---|---|---|---|---|
| pRS1/IRBPe/RS1 (SEQ ID NO: 19) | RS1 (SEQ ID NO: 22) | IRBPe (SEQ ID NO: 5) | RS1 (SEQ ID NO: 23) | 5'UTR (SEQ ID NO: 24) |
| pGRK1e-PDE6B (SEQ ID NO: 27) | - | GRKle (SEQ ID NO: 12) | PDE6B (SEQ ID NO: 7) | 5'UTR (SEQ ID NO: 24) |
| pGa1X2-PDE6B (SEQ ID NO: 32) | - | Ga1X2 (SEQ ID NO: 30) | PDE6B (SEQ ID NO: 7) | 5'UTR (SEQ ID NO: 24) |
| pGa4X2-PDE6B (SEQ ID NO: 33) | - | Ga4X2 (SEQ ID NO: 31) | PDE6B (SEQ ID NO: 7) | 5'UTR (SEQ ID NO: 24) |

| | | | | |
|---|---|---|---|---|
| ** As a spacer between the enhancer and promoter sequence, "gtcgac" (SEQ ID NO: 25) was used for the vector of SEQ ID NO: 19, and "aagaggtcgac" (SEQ ID NO: 34) was used for the vectors of SEQ ID NOs: 27, 32, and 33.* ** The coding sequence of Gaussia Luciferase, which is linked downstream of 5'UTR, was as follows:* "atgggagtcaaagttctgtttgccctgatctgcatcgctgtggccgaggccaagccca ccgagaacaacgaagacttcaacatcgtggccgtggccagcaacttcgcgaccacggat ctcgatgctgaccgcgggaagttgcccggcaagaagctgccgctggaggtgctcaaaga gctggaagccaatgcccggaaagctggctgcaccaggggctgtctgatctgcctgtccc acatcaagtgcacgcccaagatgaagaagttcatcccaggacgctgccacacctacgaa ggcgacaaagagtccgcacagggcggcataggcgaggcgatcgtcgacattcctgagat tcctgggttcaaggacttggagcccctggagcagttcatcgcacaggtcgatctgtgtg tggactgcacaactggctgcctcaaagggcttgccaacgtgcagtgttctgacctgctc aagaagtggctgccgcaacgctgtgcgacctttgccagcaagatccagggccaggtgga caagatcaagggggccggtggtgactaa"(SEQ ID NO: 41). | | | | |

An experiment was performed to confirm changes in expression in HEK293FT, 661W and rMC-1 cells according to the method described in Example 3. The results for each cell are shown in FIG. 6B to FIG. 6D, and it was confirmed that the most improved expression level was shown when the enhancer in which the Ga1 sequence was repeated twice was used.

### Example 8: Confirmation of Expression Improvement According to polyA Signal Sequence Replacement

The polyA signal sequence was replaced in the expression system of Example 7. More specifically, the PCK6 AS1 gene-derived polyA signal sequence attached to the 3' end of the Gaussia Luciferase coding sequence was replaced with a Bovine Growth Hormone (BGH) gene-derived polyA signal sequence.

Specific vector designs are shown in Table 6 and FIG. 7A.

**[Table 6]**

| | **Promoter** | **Enhancer** | **Promoter** | **5'UTR** | **polyA** |
|---|---|---|---|---|---|
| pRS1/IRBPe/RS1 (SEQ ID NO: 19) | RS1(SEQ ID NO: 22) | IRBPe (SEQ ID NO: 5) | RS1 (SEQ ID NO: 23) | 5'UTR (SEQ ID NO: 24) | PCK6 AS1 (SEQ ID NO: 35) |
| pGRK1e-PDE6B (SEQ ID NO: 27) | - | GRKle (SEQ ID NO: 12) | PDE6B (SEQ ID NO: 7) | 5'UTR (SEQ ID NO: 24) | PCK6 AS1 (SEQ ID NO: 35) |
| pGa1X2-PDE6B (SEQ ID NO: 32) | - | Ga1X2 (SEQ ID | PDE6B (SEQ ID | 5'UTR (SEQ ID | PCK6 AS1 |
| | | NO: 30) | NO: 7) | NO: 24) | (SEQ ID NO: 35) |
| pGa1X2-PDE6B-BGH (SEQ ID NO: 40) | - | Ga1X2 (SEQ ID NO: 30) | PDE6B (SEQ ID NO: 7) | 5'UTR (SEQ ID NO: 24) | Bovine Growth Hormone (BGH) (SEQ ID NO: 36) |

| | | | | | |
|---|---|---|---|---|---|
| ** As a spacer between the enhancer and promoter sequence, "gtcgac" (SEQ ID NO: 25) was used for the vector of SEQ ID NO: 19, and "aagaggtcgac" (SEQ ID NO: 34) was used for the vectors of SEQ ID NOs: 27, 32, and 40.* ** The coding sequence of Gaussia Luciferase, which is linked downstream of 5'UTR, was as follows:* "atgggagtcaaagttctgtttgccctgatctgcatcgctgtggccgaggccaagccca ccgagaacaacgaagacttcaacatcgtggccgtggccagcaacttcgcgaccacggat ctcgatgctgaccgcgggaagttgcccggcaagaagctgccgctggaggtgctcaaaga gctggaagccaatgcccggaaagctggctgcaccaggggctgtctgatctgcctgtccc acatcaagtgcacgcccaagatgaagaagttcatcccaggacgctgccacacctacgaa ggcgacaaagagtccgcacagggcggcataggcgaggcgatcgtcgacattcctgagat tcctgggttcaaggacttggagcccctggagcagttcatcgcacaggtcgatctgtgtg tggactgcacaactggctgcctcaaagggcttgccaacgtgcagtgttctgacctgctc aagaagtggctgccgcaacgctgtgcgacctttgccagcaagatccagggccaggtgga caagatcaagggggccggtggtgactaa"(SEQ ID NO: 41). | | | | | |

An experiment was performed to confirm changes in expression in HEK293FT, 661W and MIO-M1 cells according to the method described in Example 3. The results for each cell are shown in FIG. 7B to FIG. 7D, and it was confirmed that the most improved expression level was shown when the BGH polyA signal sequence was used.

### Example 9: AAV Vector Construction and Virus Production

A combination of the GRK1 enhancer and the PDE6B promoter, which showed excellent expression ability in Example 7, was inserted into the AAV vector. More specifically, the enhancer and the PDE6B promoter were sequentially inserted between the ITR sequences located on both sides, and the mCherry coding gene and the human beta globin polyA sequence were inserted at the end of the promoter 3'. The completed vector was packaged (Vectorbuilder) into adeno-associated virus serotype 8 and used for subsequent *in vivo* experiments.

AAV was constructed using a three-plasmid transfection system that does not use Helper virus using HEK293 cells, pHelper (#6230, AAVpro Helper Free System, TAKARA BIO INC) to replace the role of Helper virus, pAAV-Promoter-mCherry to express the mCherry fluorescent protein constructed in Example 8, and pRep2-Cap8 vector for packaging AAV serotype8 capsid. After AAV packaging in HEK293 cells, high-purity AAV was produced by purification through CsCl2 ultracentrifugation, and the genomic titer was measured using Primer-F: CACTACGACGCTGAGGTCAA(SEQ ID NO: 37), Primer-R: TAGTCCTCGTTGTGGGAGGT (SEQ ID NO: 38).

### Example 10: In vivo AAV injection and Immunofluorescence

Mice (C57BL/6J, DBL) were 8-week-old males and were raised in the SPF facility. The mice were injected with 4.5x10¹⁰ vg/1.5µl of AAV using a NanoFil syringe (World Precision Instruments Inc., Sarasota, Flt., USA) equipped with a 35-gauge needle using intravitreal injection and subretinal injection.

Four weeks after the virus injection, the mice were euthanized under anesthesia to extract the eyeball, and the expression of the mCherry fluorescent protein gene in the retina was confirmed using scanning laser ophthalmoscopy (SLO). After the retina was separated from the extracted eyeball, retinal sections were stained with primary anti-mCherry antibody (PA5-34974, Invitrogen) and secondary anti-mCherry antibody (Donkey anti-Rabbit IgG (H+L) Highly Cross-Adsorbed Secondary Antibody, Alexa Fluor^{™} 568, A10042, Invitrogen), and the distribution of mCherry protein in the retina and intensity were imaged by Leica DMi8 (Leica). The expression intensity of mCherry was quantified using ImageJ software, and the collected data were statistically analyzed using the Kruskal-Wallis test implemented in the SPSS statistical program (version 22.0 for Windows; IBM Corp., Armonk, NY). Post-hoc verification was conducted using Mann-Whitney U test.

The results of using intravitreal injection are shown in FIG. 8B, and the results of using subretinal injection are shown in FIGS. 8C and 9.

As can be seen in FIG. 8B, the expression system (denoted as Ga1X2P in FIG. 8) having a combination of the Ga1X2 enhancer and the PDE6B promoter showed an improved protein expression level compared to the existing patent model (denoted as RIR in FIG. 8) and GRKle (denoted as GRK1eP in FIG. 8). According to FIG. 8C and FIG. 9, the same result was shown even in the subretinal injection, and it was confirmed that when the gene expression was quantified, the expression amount was about 2.6 times or more compared to the existing patent model.

From the above description, those skilled in the art to which the present disclosure pertains will understand that the present disclosure may be implemented in other specific forms without changing the technical spirit or essential features of the present disclosure. In this regard, it should be understood that the embodiments described above are exemplary and not restrictive in all aspects. The scope of the present disclosure should be construed as including within the scope of the present disclosure all changes or modifications derived from the meaning and scope of the claims to be described below and the equivalent concept thereof rather than the detailed description.

## Claims

1. A promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7.

2. The promoter of claim 1, wherein the promoter is a retinal-specific promoter.

3. The promoter of claim 1, wherein the promoter comprises a nucleotide sequence having the sequence of SEQ ID NO: 6 or SEQ ID NO: 7.

4. An expression cassette comprising: a promoter of 100 to 400 nucleotides in length comprising as a core nucleotide sequence a nucleotide sequence having at least 90% identity to the sequence of SEQ ID NO: 6 or SEQ ID NO: 7, or the sequence of SEQ ID NO: 6 or SEQ ID NO: 7; and a nucleotide encoding a polypeptide operably linked thereto.

5. The expression cassette of claim 4, further comprising any one enhancer selected from the group consisting of SEQ ID NOs: 12 to 18 and 28 to 31.

6. The expression cassette of claim 4, wherein the promoter comprises a nucleotide sequence having the sequence of SEQ ID NO: 6 or SEQ ID NO: 7.

7. The expression cassette of claim 4, further comprising any one 5'UTR selected from the group consisting of SEQ ID NO: 8, SEQ ID NO: 9, and SEQ ID NO: 24.

8. The expression cassette of claim 4, wherein the polypeptide is a therapeutic protein or a reporter protein.

9. A vector comprising expression cassette according to any one of claims 4 to 8.

10. The vector of claim 9, wherein the vector is a viral vector or a non-viral vector.

11. The vector of claim 10, wherein the viral vector is any one selected from the group consisting of a Moloney murine leukemia virus vector (MoMLV), murine stem cell virus (MSCV), spleen focus-forming virus (SFFV), myeloproliferative sarcoma virus (MPSV) or SNV, a lentiviral vector (e.g., derived from human immunodeficiency virus (HIV), simian immunodeficiency virus (SIV), feline immunodeficiency virus (FIV), bovine immunodeficiency virus (BIV) or equine infectious anemia virus (EIAV)), an adenovirus (Ad) vector, an adeno-associated virus (AAV) vector, a simian virus 40 (SV-40) vector, a bovine papilloma virus vector, an Epstein-Barr virus vector, a herpes virus vector, a vaccinia virus vector, a Harvey murine sarcoma virus vector, a murine mammary tumor virus vector, an Anellovirus vector, and a Rous sarcoma virus vector.

12. The vector of claim 11, wherein the viral vector is an adeno-associated virus (AAV) vector.

13. The vector of claim 10, wherein the non-viral vector is any one selected from the group consisting of lipid nanoparticles (LNPs), polymer nanoparticles (PNPs), exosomes, and virus-like particles (VLPs).

14. A virus particle comprising the vector according to claim 9.

15. An adeno-associated virus (AAV) particle comprising the vector according to claim 9 and an AAV-derived capsid.

16. A cell transformed with the expression cassette according to any one of claims 4 to 8.

17. The cell of claim 16, wherein the cell is a photoreceptor cell.

18. A pharmaceutical composition comprising the expression cassette according to any one of claims 4 to 8; and a pharmaceutically acceptable excipient.

19. A pharmaceutical composition for preventing or treating an eye disease, comprising the expression cassette according to any one of claims 4 to 8.

20. A method for enhancing expression of a target gene in the retina, comprising administering the expression cassette of any one of claims 4 to 8 to a subject in need thereof.
